# EUROPEAN PATENT APPLICATION

(11) **EP 4 008 794 A1**
(43) Date of publication of application: **08.06.2022**
(21) Application number: 19940321.3
(22) Date of filing: 02.08.2019
(51) Int. Cl.: C12Q 1/6851, G01N 33/53

(54) **ANALYSIS METHOD AND KIT**

(71) Applicant: KABUSHIKI KAISHA TOSHIBA, Minato-ku Tokyo 105-0023 (JP)
(72) Inventor: INADA, Mika, Tokyo (JP); HASHIMOTO, Koji, Tokyo (JP); ITO, Keiko, Tokyo (JP)
(74) Representative: Moreland, David
(86) International application number: PCT/JP2019/030607
(87) International publication number: WO 2021/024332

(57) **Abstract**

According to the embodiment, analytical method for determining the presence/absence of the contraction of the prostatic cancer in subjects is provided. The method comprises quantifying hsa-miR-92a-3p in a sample originated from a subject.

SEQUENCE LISTING

<110>  Kabushiki Kaisha Toshiba

<120>  Analytical method and Kit

<130>  17S0179PCT

<160>  15

<170>  PatentIn version 3.5

<210>  1
<211>  22
<212>  RNA
<213>  human

<400>  1
uauugcacuu gucccggccu gu                                                22

<210>  2
<211>  26
<212>  DNA
<213>  artificial

<220>
<223>  RT primer

<400>  2
ggaggcgaca cgagttctac aggccg                                            26

<210>  3
<211>  79
<212>  DNA
<213>  artificial

<220>
<223>  EL primer

<400>  3
ggctgtgcag agataggtgg tacgaagcag ccttctcggc tcggctatct acgcgttaag      60

cggggtattg cacttgtcc                                                    79

<210>  4
<211>  28
<212>  DNA
<213>  artificial

<220>
<223>  RT primer

<400>  4
ggcgccgaaa caatattcct acaggccg                                          28

<210>  5
<211>  77
<212>  DNA
<213>  artificial

<220>
<223>  EL primer

<400>  5
gatctagaag gccgccagtc gttcagccta cggccgttgt catccgtagc aggacgctca      60

gggtattgca cttgtcc                                                    77


<210>  6
<211>  27
<212>  DNA
<213>  artificial

<220>
<223>  RT oprimer

<400>  6
ctgcaacgtt gaacatgcga caggccg                                         27


<210>  7
<211>  80
<212>  DNA
<213>  artificial

<220>
<223>  EL primer

<400>  7
ttcgtgcaga tggctatgcg gctagaatac ggcacgactg ctgatgtgtc tgatagcggc      60

acggggtatt gcacttgtcc                                                 80


<210>  8
<211>  39
<212>  DNA
<213>  artificial

<220>
<223>  FIP primer

<400>  8
gccgagaagg ctgcttcgta ggctgtgcag agataggtg                            39


<210>  9
<211>  40
<212>  DNA
<213>  artificial

<220>
<223>  BIP primer

<400>  9
tcggctatct acgcgttaag cgggaggcga cacgagttct                           40


<210>  10
<211>  16
<212>  DNA

<213>  artificial

<220>
<223>  LB primer

<400>  10
acttgtcccg gcctgt                                                        16


<210>  11
<211>  17
<212>  DNA
<213>  artificial

<220>
<223>  LB primer

<400>  11
ttgtcccggc ctgtaga                                                       17


<210>  12
<211>  38
<212>  DNA
<213>  artificial

<220>
<223>  FIP primer

<400>  12
aacggccgta ggctgaacgg atctagaagg ccgccagt                                38


<210>  13
<211>  42
<212>  DNA
<213>  artificial

<220>
<223>  BIP primer

<400>  13
gtcatccgta gcaggacgct caggcgccga aacaatattc ct                           42


<210>  14
<211>  42
<212>  DNA
<213>  artificial

<220>
<223>  FIP primer

<400>  14
gcagtcgtgc cgtattctag cccttcgtgc agatggctat gc                           42


<210>  15
<211>  41
<212>  DNA
<213>  artificial

<220>

<223>  BIP primer

<400>  15
tgatgtgtct gatagcggca cgctgcaacg ttgaacatgc g                                41

## Description

### Technical Field

Embodiments described herein relate generally to an analytical method and a kit.

### Background Art

Recently, the relationship between microRNA (miRNA) and diseases is getting attention. The miRNA has a function to regulate a gene expression, and it is reported that the type and the amount of expression thereof change from an early stage in various kinds of diseases. That is, in a patient with a certain disease, the quantity of a particular miRNA increases or decreases as compared to the case of a normal persons. Therefore, examination of the quantity of the miRNA in a sample collected from a subject can be measures to know whether the subject patient contracts the certain disease.

### Summary of Invention

### Technical Problem

One of embodiments described herein aims to provide an analytical method that can easily determine the presence or absence of contraction of cancer, in particular, prostatic cancer in subjects.

### Solution to Problem

According to the embodiment, analytical method for determining the presence/absence of the contraction of the prostatic cancer in subjects is provided. The method comprises quantifying hsa-miR-92a-3p in a sample originated from a subject.

### Brief Description of Drawings

FIG. 1 is a flowchart showing an example of the analytical method of the first embodiment.
FIG. 2 is a flowchart showing an example of the quantification process of the embodiment.
FIG. 3 is a schematic diagram showing an example of the quantification process of the embodiment.
FIG. 4 is a flowchart showing an example of the analytical method of the first embodiment.
FIG. 5 is a flowchart showing an example of the analytical method of the second embodiment.
FIG. 6 is a flowchart showing an example of the analytical method of the third embodiment.
FIG. 7 is a graph showing experimental results in example 1.
FIG. 8 is a graph showing experimental results in example 1.
FIG. 9 is a graph showing experimental results in example 2.
FIG. 10 is a graph showing experimental results in example 2.
FIG. 11 is a graph showing experimental results in example 3.
FIG. 12 is a graph showing experimental results in example 4.

### Mode for Carrying Out the Invention

Analytical method and kits of the embodiments will be described below with reference to the accompanying drawings.

### -First Embodiment

### (Analytical method)

The analytical method of the embodiment is a method for determining the presence/absence of contraction of prostatic cancer in a subject.

The subject may be an animal to be subjected to the analysis, which is an animal providing a sample which will be described below. The subject may be an animal contracting some kind of diseases or may be a normal animal. For example, the subject may be an animal which may be contracting prostatic cancer, an animal which may have been contracted prostatic cancer or the like.

The subject may preferably be a human, or the subject may be some other animal. Some other animal may be, for example, a mammal, such as a primate such as a monkey, a rodent such as a mouse, rat or guinea pig, a companion animal such as dog, cat or rabbit, or a domestic animal such as horse, cow or pigs, or a displayed animal or the like.

The prostatic cancer according to the embodiment refers to a malignant tumor (neoplasm) that begins to develop in prostate. The prostatic cancer includes those generally referred to as "prostate cancer", "prostatic tumor" or "cancer of prostate". Further, the prostatic cancer according to the embodiment includes that of any stage of the disease, that is, for example, a state that a cancer remains within prostate, a state that the cancer reaches the surrounding tissues, a state that the cancer metastasizes in lymph node, a state that the cancer metastasizes to a farther remote organ, and the like.

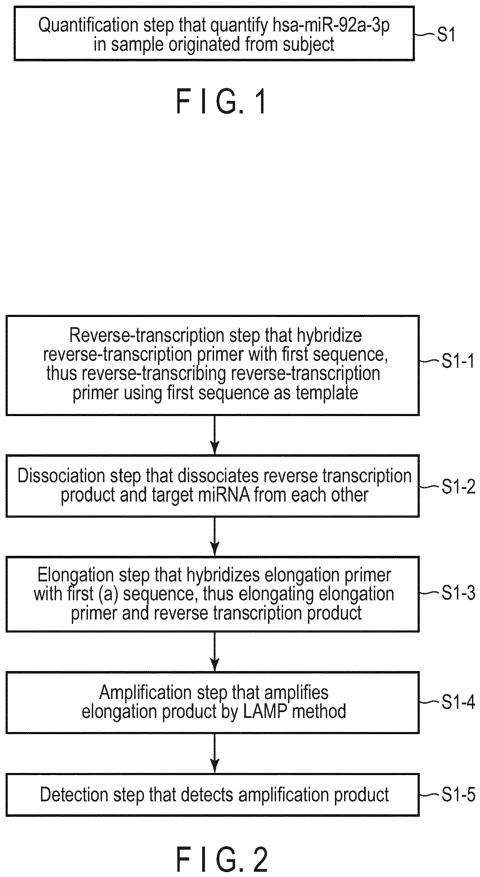

The analytical method comprises quantifying hsa-miR-92a-3p in a sample originated from a subject (quantification step (S1)), as shown in FIG. 1.

A sample originated from the may preferably be serum. The sample may be some other body fluid, for example, blood, plasma, stroma liquid, urine, feces, sweat, saliva, oral mucosa, intranasal mucous membrane, pharyngeal mucosa, sputum, lymph fluid, cerebrospinal fluid, tears, mother's milk, amniotic fluid, semen or the like. Or, the sample may be cultured tissues or cells sampled from a subject, or a supernatant thereof. Or it may be an established cell line from the cultured cells.

The method of collecting a sample may be a general method based on the type of the sample. The collected sample may be, for example, pre-treated with any well-known means so as to set it, for example, to be in a condition not to inhibit a reverse transcription, elongation and amplification reactions, which will be described below or to set it in a condition more suitable for these reactions. The pre-treatment is, for example, slicing, homogenizing, centrifuging, precipitation, extraction and/or separation or the like.

For example, the extraction may be carried out with use of a commercially available nucleic acid extraction kit. Or, the extraction may be carried out without using a commercially available kit, but by, for example, diluting the material with a buffer solution, subjecting it to a heat-treatment at 80 to 100°C and centrifuging, and then collecting its supernatant.

The hsa-miR-92a-3p is an miRNA having the following sequence:
UAUUGCACUUGUCCCGGCCUGU (SEQ ID NO. 1).

Note that the hsa-miR-92a-3p may as well be referred to as "target miRNA" hereinafter. Further, the sequence of SEQ ID NO. 1 may as well be referred to as "the first sequence".

The quantification of a target miRNA can be carried out by a general method that can quantify RNA. For example, reverse-transcribing, elongating and/or amplifying of the target miRNA, or a PCR method, a qPCR method, a LAMP method or the like, a turbidity or an absorbency measurement method, a fluorometry, or an electrochemical measurement method or a combination of any of these, or the like can be used.

When using the qPCR method, for example, a commercially available kit can be used for quantification. Examples of the commercially available kit is TaqMan (registered trademark) Advanced miRNA Assays (a product of ThermoFischer, ID: 477827_mir), miRCURY LNA miRNAPCR Assays (a product in Kia Gene, Catalog No. YP02103132) and the like. Further, quantification can be carried out by using SYBR (registered trademark) Green qPCR microRNA detection system (a product of Origin Technologies) and a primer which specifically amplifies a target miRNA.

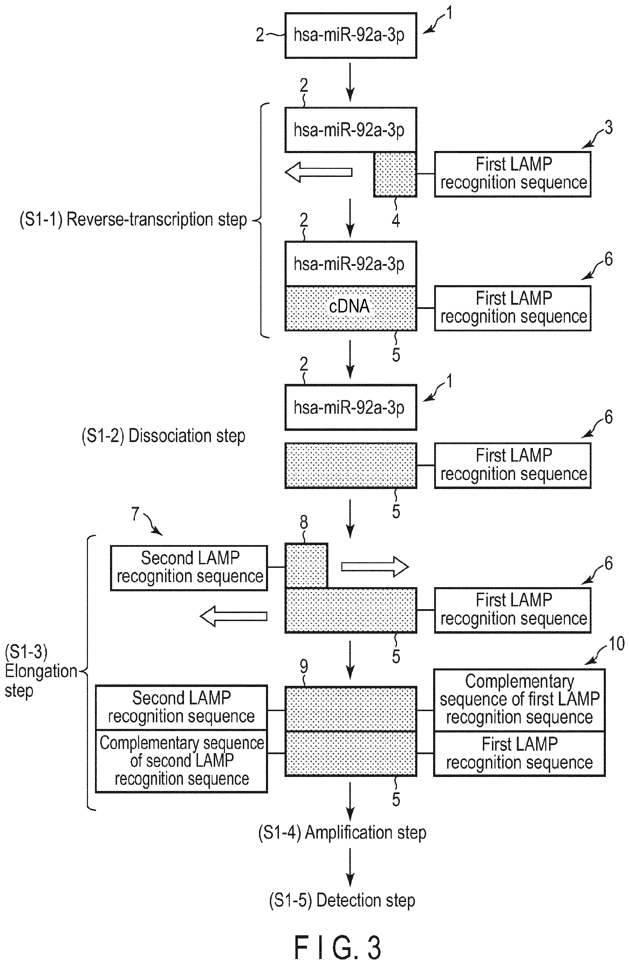

A preferable quantification step (S1) is a method carried out by specifically reverse-transcribing and elongating a target miRNA, amplifying the elongated product by the LAMP method, and detecting the amplified product. An example of such a quantification step (S1) will be described as follows. The quantification step (S1) includes the following steps shown in, for example, FIGS. 2 and 3.
(S1-1) a reverse-transcription step for hybridizing, a first primer portion 4 included in a reverse-transcription primer 3 (to be referred to also as "RT primer" hereinafter) and a first sequence 2 of the target miRNA1, wherein the first primer portion 4 is hybridizable with the first sequence 2, and a reverse-transcription primer 3 further contains a first LAMP recognition sequence, reverse-transcribing the first sequence 2, and obtaining a reverse transcription product 6 containing a first (a) sequence 5 (cDNA of a target miRNA1);
(S1-2) a dissociation step for dissociating the reverse transcription product 6 and the target miRNA1 from each other;
(S1-3) an elongation step for hybridizing a second primer portion 8 included in an elongation primer 7 (to be referred to also as "RT primer" hereinafter) and the first (a) sequence 5 included in the reverse transcription product 6, wherein the second primer portion 8 is hybridizable with the first (a) sequence 5, and the elongation primer 7 further contains the second LAMP recognition sequence, elongating the EL primer 7 and the reverse transcription product 6 using these as templates with respect to each other, and obtaining an elongation product 10 containing a complementary sequence 9 of the first (a) sequence 5 (that is, a DNA sequence corresponding to the first sequence); and
(S1-4) an amplification step for amplifying the elongation product 10 by the LAMP reaction, and obtaining an amplification product; and
(S1-5) a detection step for detecting the obtained amplification product.

The first primer portion 4 of the RT primer 3 is a nucleic acid sequence which hybridizes with the first sequence 2 and acts as a primer which reverse-transcribe the first sequence 2 to generate cDNA. The first primer portion 4 comprises, for example, at least five continuous base sequence containing a 3' end of the first sequence 2.

The second primer portion 8 of the EL primer 7 is a nucleic acid sequence which hybridizes with the first (a) sequence 5 and acts as a primer for generate a complementary sequence thereof. The second primer portion 8 comprises, for example, at least five continuous base sequence containing the 3' end of the first (a) sequence 5.

The first primer portion 4 and the second primer portion 8 may be constituted by only DNA or may contain LNA and/or PNA. The more amounts of these contained, the stronger the bonding strength of the hybridization can be obtained. Therefore, the number of LNAs and/or PNAs may be determined according to desired Tm value with respect to the sequence to be hybridized. For example, when LNA and/or PNA are contained, the reverse transcription step (S1-1) and the elongation step (S1-3) can be carried out at higher temperature, and thus nonspecific bonds can be inhibited. As a result, the target miRNA can be reverse-transcribed and elongated with higher accuracy.

The first LAMP recognition sequence and the second LAMP recognition sequence are nucleic acid base sequences containing a sequence to which a LAMP primer is bonded (that is, recognition sequence) in the amplification step (S1-4). The recognition sequence may be generally that used in the LAMP primer. For example, the first LAMP recognition sequence may contain a B1 sequence, a B2 sequence (and optionally an LB sequence) and a B1 sequence in the order from a first primer portion 4 side towards the 5' end. The second LAMP recognition sequence may contain an F1 sequence, an F2 sequence (and optionally, an LB sequence) and an F1 sequence in the order from the second primer portion 8 side towards the 5' end.

However, it is preferable that the first LAMP recognition sequence and the second LAMP recognition sequence contain recognition sequences in combination of the following (1) to (7). Here, the order of each recognition sequence listed below is that from the first primer portion 4 or second primer portion 8 side towards the 5' end. The symbol "c" means a complementary sequence of the sequence described just before that. For example, the "LBc sequence" means a complementary sequence of the LB sequence. A "dummy sequence" is a nucleic acid sequence containing a base sequence different from a first (a) sequence, an F2 sequence, an F1 sequence, an LF sequence, a B1 sequence, a B2 sequence, an LB sequence and the complementary sequence thereof.
(1) The first LAMP recognition sequence contains a B2 sequence, and
   the second LAMP recognition sequence contains a B1c sequence, an F1 sequence and an F2 sequence. (In this case, at least a part of the complementary sequence 9 of the first (a) sequence is defined as an LB sequence.)
(2) The first LAMP recognition sequence contains an LBc sequence and a B2 sequence, and
   the second LAMP recognition sequence contains a B1c sequence, an F1 sequence and an F2 sequence.
(3) The first LAMP recognition sequence contains a dummy sequence and a B2 sequence, and
   the second LAMP recognition sequence contains B1c sequence, F1 sequence and F2 sequence.
(4) The first LAMP recognition sequence contains a B1 sequence and a B2 sequence, and
   the second LAMP recognition sequence contains an F1 sequence, an LFc sequence and an F2 sequence.
(5) The first LAMP recognition sequence contains a B2 sequence, and
   the second LAMP recognition sequence contains an F1 sequence, an LFc sequence and an F2 sequence.
(6) The first LAMP recognition sequence contains an LBc sequence and a B2 sequence, and
   the second LAMP recognition sequence contains an F1 sequence and an F2 sequence.
(7) The first LAMP recognition sequence contains a B1 sequence and a B2 sequence, and
   the second LAMP recognition sequence contains an LFc sequence and an F2 sequence.

With the combinations listed above, the target miRNA can be more specifically reverse-transcribed and elongated, and thus the accuracy of detection of prostatic cancer can be improved.

When the combination of the LAMP recognition sequences is selected as (1) above, it is preferable to use any one of reverse transcription and elongation primer sets A to C, which contains an RT primer 3 and an EL primer 7 listed in Table 1 below.

**Table 1**

| Reverse transcription and elongation primer set A | | |
|---|---|---|
| Primer | SEQ ID NO | Sequence (5'-3') |
| RT | 2 | GGAGGCGACACGAGTTCTACAGGCCG |
| EL | 3 | GGCTGTGCAGAGATAGGTGGTACGAAGCAGCCTTCTCGGCTCGGCTAT CTACGCGTTAAGCGGGGTATTGCACTTGTCC |

| Reverse transcription and elongation primer set B | | |
|---|---|---|
| Primer | SEQ ID NO | Sequence (5'-3') |
| RT | 4 | GGCGCCGAAACAATATTCCTACAGGCCG |
| EL | 5 | GATCTAGAAGGCCGCCAGTCGTTCAGCCTACGGCCGTTGTCATCCGTAG CAGGACGCTCAGGGTATTGCACTTGTCC |

| Reverse transcription and elongation primer set C | | |
|---|---|---|
| Primer | SEQ ID NO | Sequence (5'-3') |
| RT | 6 | CTGCAACGTTGAACATGCGACAGGCCG |
| EL | 7 | TTCGTGCAGATGGCTATGCGGCTAGAATACGGCACGACTGCTGATGTGT CTGATAGCGGCACGGGGTATTGCACTTGTCC |

| | | |
|---|---|---|
| *Bold letters indicate first primer or second primer | | |

A spacer sequence may exist between the first primer portion 4 and the first LAMP recognition sequence of the RT primer 3, between the second primer portion 8 and the second LAMP recognition sequence of the EL primer 7 and/or between a respective pair of recognition sequences contained in each LAMP recognition sequence. The spacer sequence is a nucleic acid sequence different from the first (a) sequence, each recognition sequence and complementary sequences thereof, which does not adversely affect the amplification reaction of the elongation product, which will be described below. The spacer sequence may be constituted by only DNA or may contain LNA and/or PNA. For example, the spacer sequence is of 1 base to 16 bases, and it is preferable that it may be a poly-T-sequence, a poly-A sequence or the like.

The reverse transcription production step (S1-1) is carried out, for example, by adding the RT primer 3, reverse transcriptase, salt and substrates such as deoxynucleoside triphosphate (dNTP) and the like (as needed, a thickener, pH-adjustment buffer material, a surfactant as reaction reagents, ions which enhance annealing specificity and/or ions serving as a cofactor of reverse transcriptase) and the like, to the sample. Usable examples of reverse transcriptase are M-MuLV reverse transcriptase, AMV reverse transcriptase, transcripter reverse transcriptase, SuperScript (registered trademark) transcripter reverse transcriptase and MultiScribe reverse transcriptase. It is preferable to maintain the temperature at, for example, approximately 10°C to 55°C.

The dissociation step (S1-2) can be carried out by, for example, heating the reaction solution to 80°C to 100°C.

The elongation step (S1-3) is carried out by, for example, adding the EL primer 7, DNA polymerase, salt and substrates such as dNTP and the like (as needed, a thickener, pH-adjustment buffer material, a surfactant and ions) and the like to a solution containing the reverse transcription product 6. It is preferable to maintain the temperature at approximately 10°C to 80°C.

The amplification step (S1-4) is carried out using the LAMP method. This step includes adding a LAMP primer set, strand-displaced DNA polymerase, salt and substrates such as dNTP (as needed, a thickener, pH-adjustment buffer, a surfactant and ions) and the like, to a solution containing an elongation product 10, and maintaining the solution under conditions of an isothermal amplification reaction.

The type and the sequence of LAMP primer set primer set are selected according to the sequences of the first LAMP recognition sequence and the second LAMP recognition sequence. For example, LAMP primer set primer set contains an FIP primer and a BIP primer, correspond to the sequences of the first LAMP recognition sequence and the second LAMP recognition sequence. If necessary, an F3 primer, a B3 primer and/or a loop primer such as LF primer or an LB primer or the like may be contained.

When the combination of the LAMP recognition sequences is selected as (1) above, for example, one of LAMP primer sets A to C listed in Table 2 below can be used as LAMP primer set primer set. Note that, for the LB primer of the LAMP primer set A, one of SEQ ID NOS. 10 and 11 can be used.

**Table 2**

| LAMP primer set A | | |
|---|---|---|
| Primer | SEQ ID NO | Sequence (5'-3') |
| FIP | 8 | GCCGAGAAGGCTGCTTCGTAGGCTGTGCAGAGATAGGTG |
| BIP | 9 | TCGGCTATCTACGCGTTAAGCGGGAGGCGACACGAGTTCT |
| LB | 10 | ACTTGTCCCGGCCTGT |
| LB | 11 | TTGTCCCGGCCTGTAGA |

| LAMP primer set B | | |
|---|---|---|
| Primer | SEQ ID NO | Sequence (5'-3') |
| FIP | 12 | AACGGCCGTAGGCTGAACGGATCTAGAAGGCCGCCAGT |
| BIP | 13 | GTCATCCGTAGCAGGACGCTCAGGCGCCGAAACAATATTCCT |
| LB | 10 | ACTTGTCCCGGCCTGT |

| LAMP primer set C | | |
|---|---|---|
| Primer | SEQ ID NO | Sequence (5'-3') |
| FIP | 14 | GCAGTCGTGCCGTATTCTAGCCCTTCGTGCAGATGGCTATGC |
| BIP | 15 | TGATGTGTCTGATAGCGGCACGCTGCAACGTTGAACATGCG |
| LB | 10 | ACTTGTCCCGGCCTGT |

According to the above-discussed steps, the target miRNA can be more specifically reverse-transcribed, elongated and amplified. Particularly, in the case of using the primers shown in Tables 1 and 2, the specificity in the reverse transcription, elongation and amplification improves. Therefore, it is possible to detect the presence/absence of contraction of prostatic cancer at higher sensitivity.

The detection step (S1-5) can be carried out by a general method for detecting nucleic acid. For example, a signal of the turbidity, an optical signal or an electrochemical signal and the like can be used for the detection step.

When using the turbidity for example, the turbidity of the reaction solution, which increases depending on the presence of the amplification product or the amplification reaction, the amount of change in turbidity or the rise time of the turbidity are detected. The detection can be carried out by, for example, using a turbidimeter or a spectrophotometer or visual inspection or the like.

When using the optical signal, for example, the amplification reaction may be carried out in the presence of a labeling substance (a fluorescent reagent, intercalator or the like) which produces an optical signal which changes according to the presence of the amplification product or the amplification reaction, and the amount, the amount of change or the rise time of the optical signal may be detected. The detection can be carried out by, for example, using one of the well-known optical sensors, or visual inspection or the like.

When using the electrochemical signal, the amplification reaction may be carried out in the presence of a labeling substance (a redox probe or the like) which produces the electrochemical signal which changes according to the increase in the amplification product, and the amount, the amount of change and the rise time of the electrochemical signal may be detected. The detection can be carried out by, for example, using am electrochemical detection device with an electrode.

The electrochemical detection device includes, for example, a chip. The chip comprises a substrate comprising an electrode on one surface. The electrode may preferably be, for example, gold because of its high sensitivity. When a solution containing the amplification product is brought in on the one surface, the electrode is brought into contact with the solution, and thus an electrochemical signal can be detected from the labeling substance present there.

Above described detections of the turbidity, optical signal and electrochemical signal and the like, may be carried out at a particular time point after the initiation of the amplification reaction, or may be carried out over time. The detection over time may mean continuous detection or detection at a plurality of time points with predetermined time intervals.

Further, for the detection of the amplification product, for example, a nucleic acid probe containing at least a part of the sequence of a target miRNA, cDNA of the target miRNA, the elongation product 11 or a complementary sequence thereof may be used. By detecting the hybridization between such a nucleic acid probe and amplification product, the amplification product can be detected.

As described above, the amount, the amount of change, the rise time of each signal obtained in the detection step (S1-5) reflect the amount of the amplification product. From these data items, the amount of the target miRNA originally present in the sample can be calculated, that is, quantified.

For example, the greater, the amount of the target miRNA present in the sample, the shorter, the time for rising the change in signal can be observed. Therefore, using an analytical curve representing the relationship between the rise time and the amount of target miRNA present, the target miRNA in the sample can be quantified. The analytical curve can be prepared by measuring the rise time of the signal about a plurality of standard samples containing the target miRNA at different known concentrations. The analytical curve is compared with the results of the measurement of the rise time obtained for the sample originated from the subject. Thus, the amount of the target miRNA present in the sample can be calculated.

For example, the amount of a target miRNA present in a sample can be obtained as the number of copies of the target miRNA per unit quantity of the sample.

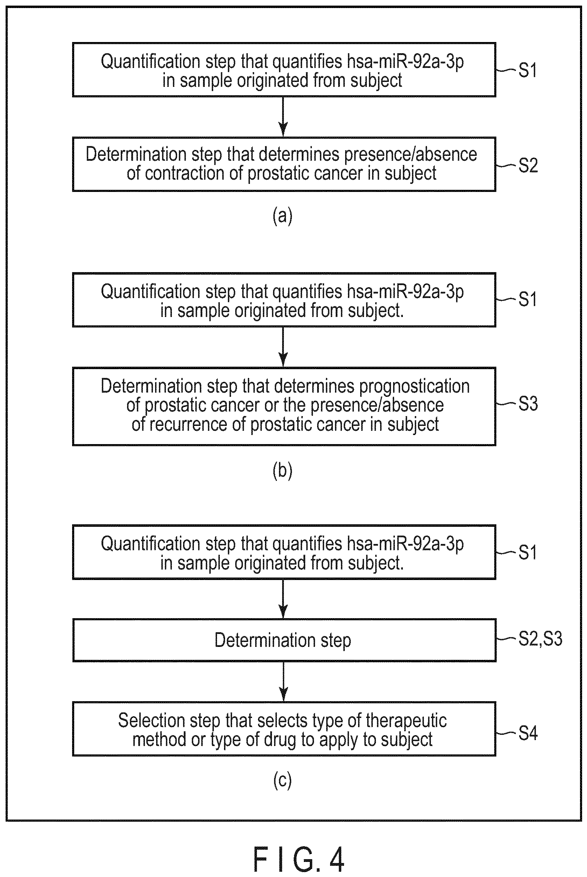

From the quantification result obtained in the quantification step (S1) according to one of the methods discussed above, the presence/absence of contraction of prostatic cancer of the subject can be determined. Such determination can be made by, for example, a determination step (S2) that can be carried out after the quantification step (S1) shown in FIG. 4, part (a).

For example, in the determination step (S2), when the amount of the target miRNA present in the subject is greater than the amount of the target miRNA in a control, it can be determined that the subject contracts prostatic cancer. The control may be a normal body. The normal body means an individual which does not contract at least prostatic cancer. It is preferable that the normal body be a healthy individual not contracting any diseases or abnormalities. Or, the control may be an individual contracting a cancer except for the prostate cancer.

Here, an individual selected as a control can be an individual different from the subject to be examined by the analytical method, but it is preferable that the individual belong to the same species, that is, if the subject is a human, it may be a human. Further, physical conditions such as the age, sex, height and weight of the control and the number of persons in the control are not particularly limited, but the physical conditions may preferably be the same or similar to those the subject to be examined by the analytical method.

The amount of the target miRNA present in the control can be that obtained using the same or similar kind of sample and method in advance before carrying out the analytical method of the embodiment. Or, the amount of the target miRNA present in the control may be a value obtained from the past findings such as of literatures and the like.

Or, when the quantification value obtained in the quantification step (S1) is higher than or equal to a predetermined threshold value, it can be determined that the subject contracts prostatic cancer. For example, the threshold value can be decided by comparing the measurements of the target miRNA quantification values of a normal individual or an individual having some other kind of cancer and an individual contracting prostatic cancer, obtained by the same method as that used in the quantification step (S1), with each other. Each "individual" may contain a plurality of individuals. The threshold value may vary according to the method employed, the type of the sample, and the measurement conditions.

Here, the determination of a subject contracting prostatic cancer also includes a determination that the subject has a high possibility of contracting prostatic cancer.

Further, the determination of a subject contracting prostatic cancer also includes determining the presence/absence of contraction of prostatic cancer of the subject as distinct from the other cancers. In other words, when determined to contract prostatic cancer in the determination step (S2), it can mean that the subject is contracting no other cancers but prostatic cancer. The other cancers include, for example, those classified as other than the prostatic cancer (malignant tumor, malignant neoplasm, carcinoma and sarcoma). For example, the other cancers include breast cancer, colon cancer, stomach cancer, lung cancer, ovarian cancer, prostate cancer, bile duct cancer, esophagus cancer, liver cancer, brain tumor, bladder cancer, sarcoma, endometrial cancer and uterus sarcoma and the like.

Further, the determination of a subject contracting prostatic cancer also includes a determination as to prognosis of the prostatic cancer or the presence/absence of the recurrence of the prostatic cancer in the subject. In this case, the subject can be, for example, a subject which have been determined to contract prostatic cancer before subjected to the analytical method, a subject which has been treated for the prostatic cancer, a subject which have been healed from prostatic cancer and/or a subject subjected to a following-up for prostatic cancer, or the like.

For example, when a result of a quantification of the target miRNA in a subject indicates that the quantification value is higher than that of the control or at a predetermined threshold or higher, it can be determined that the prognosis of prostatic cancer is poor, or prostatic cancer recurs or is highly likely to have recur in the subject. Such an analytical method includes a determination step (S3) which determines the prognosis of prostatic cancer or the presence/absence of the recurrence of the prostatic cancer in the subject from the result of the quantification after the quantification step (S1) as shown in FIG. 4, part (b).

In another embodiment, after the determination step (S2) or (S3), a type of therapeutic method or a type of drug to apply to a subject can be selected according to the result. The therapeutic method or the drug is for the treatment of prostatic cancer. The type of therapeutic method or the type of drug includes the therapeutic method, the dosage of the drug, the timing or the period thereof. Such an analytical method includes a selection step (S4) for selecting a type of therapeutic method or a type of drug to apply to a subject based on the result of the quantification after the determination step (S2) or (S3) as shown in FIG. 4, part (c).

Moreover, in another embodiment, in order to make the determination more reliable after the determination step (S2) or (S3), the subject may be subjected to a further examination for the prostatic cancer, that is, for example, cytodiagnosis and the like.

According to the analytic method of the embodiment described above, the presence/absence of contraction of prostate cancer in a subject can be determined easily by quantifying one type of miRNA (hsa-miR-92a-3p).

In particular, in the analytical method of the embodiment, serum, which can be easily collected by medical examinations and the like, can be used, and therefore prostatic cancer can be early detected. In addition, the analytical method of the embodiment can greatly reduce the physical and economic burden of the subject as compared to cytodiagnosis and the like, and the procedure is easy for laboratory technicians to perform with less load. Further, with the serum, more accurately examination can be carried out because the concentration of miRNA contained therein is stable.

### (Marker)

According to the embodiment, a marker for the detection of prostatic cancer, which contains hsa-miR-92a-3p, is provided.

For example, the marker for the detection of prostatic cancer of the embodiment can be used for the determination of the presence/absence of contraction of prostatic cancer in a subject, the determination of prognostication of prostatic cancer in a subject and the determination of the presence/absence of recurrence of prostatic cancer in a subject.

Further, the marker of the embodiment can be also used for the selection of the type of therapeutic method or the type of drug to apply to a subject. Here, the therapeutic method or drug is for the treatment of prostatic cancer.

Further, the marker of the embodiment can be also used for the detection of prostatic cancer cells in a sample.

### (Kit)

According to the embodiment, a kit for the detection of prostatic cancer is provided.

The kit comprises at least one kind of nucleic acid selected from a group consisting of a primer set (to be referred to as "the first primer set" hereinafter) to amplify hsa-miR-92a-3p, an RT primer to reverse-transcribe hsa-miR-92a-3p, an EL primer to elongate hsa-miR-92a-3p and nucleic acid probes to detect hsa-miR-92a-3p.

For example, the first primer set is LAMP primer set. For example, as the first primer set, at least one of LAMP primer sets A to C listed in Table 2 can be used.

When the kit of the embodiment includes an RT primer and an EL primer, at least one of reverse transcription and elongation primer sets A to C listed in Table 1 can be used.

The nucleic acid probe may include at least a part of sequence of hsa-miR-92a-3p or a complementary sequence thereof, or at least a part of sequence of cDNA of hsa-miR-92a-3p or a complementary sequence thereof.

The nucleic acids contained in the kit may be individually separated or combined in any combination, and contained in a container together with an appropriate carrier. The appropriate carrier is, for example, water or a buffer solution or the like. The container is, for example, a tube or a microtiter plate or the like. Further, these may be fixed to a solid phase such as a microfluidic chip or the like, to be provided.

The kit may include, in addition to the nucleic acids, a reagent used for the reverse transcription, elongation and/or amplification, an indicator used for the detection (for example, a fluorochrome such as SYBRGreen or EVAGreen, or to detect the current, a metal complex such as ruthenium hexamine or the like) and the like.

The kit for the prostatic cancer detection of the embodiment can be used for, for example, the determination of the presence/absence of contraction of prostatic cancer in a subject, the determination of prognostication of prostatic cancer in a subject and the determination of the presence/absence of recurrence of prostatic cancer in a subject, and the like. Further, the kit of the embodiment can be also used for the selection of the type of therapeutic method or the type of drug to apply to a subject. Here, the therapeutic method or drug is for treatment of prostatic cancer. Or, the kit of the embodiment can be also used for the detection of prostatic cancer cells in a sample, and the like.

According to the another embodiment, the kit for the detection of prostatic cancer is provided as a diagnostic composition or diagnostic medicine for prostatic cancer, which contains at least one type of nucleic acid selected from a group consisting of a primer set to amplify hsa-miR-92a-3p, an RT primer to reverse-transcribing hsa-miR-92a-3p, an EL primer to elongate hsa-miR-92a-3p and nucleic acid probes to detect hsa-miR-92a-3p. Further, according to the embodiment, the use of at least one type of the nucleic acids in the production of the diagnostic composition for prostatic cancer or the diagnostic medicine for prostatic cancer is also provided.

### -Second Embodiment

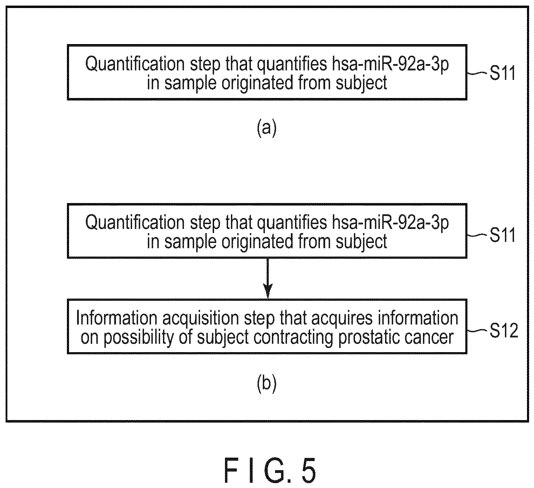

In the second embodiment, as shown in FIG. 5, part (a), an analytical method for assisting the determination of the presence/absence of contraction of prostatic cancer in a subject, which includes (a quantification step (S11)) quantifying hsa-miR-92a-3p in a sample originated from the subject, is also provided.

Here, the "assisting the determination" includes, for example, acquiring information regarding the possibility that the subject is contracting prostatic cancer. Such an analytical method includes the following steps as shown in FIG. 5, part (b).
(S11) a quantification step for quantifying hsa-miR-92a-3p in a sample originated from the subject; and
(S12) an information acquisition step for acquiring information regarding the possibility that the subject is contracting prostatic cancer.

The quantification step (S11) can be carried out by a method similar to that of the quantification step (S1). The information obtained in the information acquisition step (S12) is information regarding the amount of the target miRNA present in the sample, provided from the result of the quantification in the quantification step (S11). For example, the information is a quantification value of the amplification product or a quantification value of the target miRNA in a sample, or the like.

The information is compared with a control as described above or a predetermined threshold, to be used for the determination of the presence/absence of contraction of prostatic cancer in a sample originated from a subject, the determination of prognostication of prostatic cancer in a subject, the determination of the presence/absence of recurrence of prostatic cancer, or the selection of the type of therapeutic method or the type of drug to apply to a subject, or the like.

### -Third Embodiment

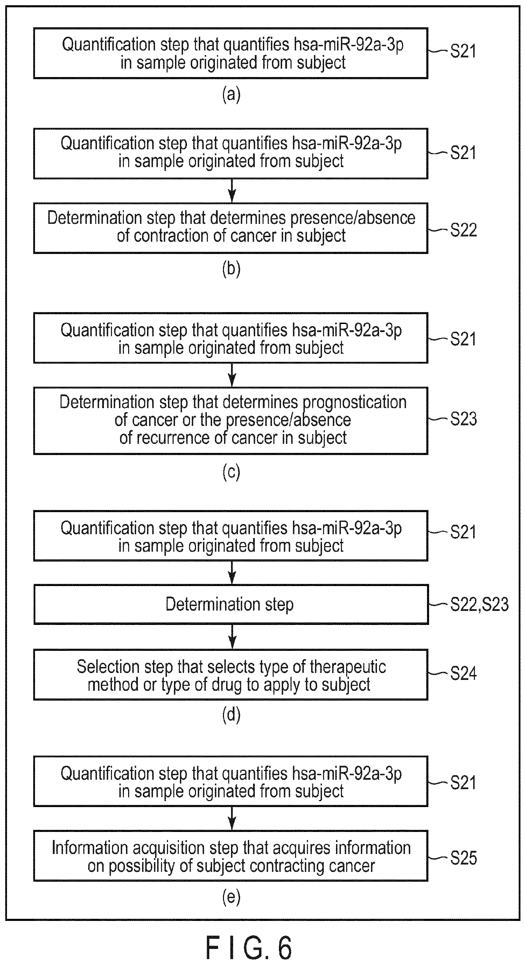

According to the third embodiment, as shown in FIG. 6, part (a), the presence/absence of contraction of cancer in a subject is determined by quantifying hsa-miR-92a-3p in a sample originated from the subject (a quantification step (S21)).

The cancer includes malignant tumor, malignant neoplasm, carcinoma and sarcoma. The cancer includes, for example, prostate cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer, uterus sarcoma and the like.

In this analytic method, the quantification step (S21) can be carried out by a method similar to above described quantification step (S1).

From the results of the quantification obtained in the quantification step (S21), the presence/absence of contraction of cancer in the subject can be determined. The determination can be carried out, for example, a determination step (S22) that can be performed after the quantification step (S21) shown in FIG. 6, part (b). For example, in the determination step (S22), when the quantification value of the target miRNA of a subject is higher than that of a control, the subject can be determined to contract cancer. The control may be a quantification value of the target miRNA in a normal body. Here, the normal body may be an individual which has not contracted cancer.

Or, when the quantification value obtained in the quantification step (S21) is at a predetermined threshold or higher, the subject may be determined to contract cancer.

Further, the determination of a subject contracting cancer also includes a determination a high possibility of the subject contracting cancer, a determination as to the prognosis of the cancer of the subject being poor, and/or the presence/absence of the recurrence of the cancer in the subject. Such an analytic method includes a determination step (S23) to determine the prognosis of the cancer in the subject or the presence/absence of the recurrence of cancer in the subject from the results of the quantification, carried out after the quantification step (S21) as shown in FIG. 6, part (c).

In this method, the determination of a subject contracting cancer includes a determination the presence/absence of contraction of cancer of the subject as distinct from the normal state. Here, the normal state means that has not contracted cancer.

Further, from the results of the determination step (S22), a type of therapeutic method or a type of drug to apply to a subject can be selected. Such an analytical method includes a selection step (S24) for selecting a type of therapeutic method or a type of drug to apply to a subject based on the result of the quantification after the determination step (S22) or (S23) as shown in FIG. 6, part (d). Moreover, after the determination step (S22), the subject may be subjected to a further examination to make the determination more reliable, or to determine the type of the cancer of the subject.

According to the analytic method of the embodiment described above, the presence/absence of contraction of cancer in a subject can be determined easily by quantifying one kind of miRNA (hsa-miR-92a-3p).

According to the third embodiment, an analytical method for assisting determination of the presence/absence of contraction of cancer in a subject, which includes quantifying hsa-miR-92a-3p in a sample originated from the subject (a quantification step (S21)), is also provided. Here, the "assisting the determination" includes, for example, an information acquiring step (S25) for acquiring information regarding the possibility that the subject is contracting cancer, as shown in FIG. 6, part (e).

According to the third embodiment, a marker for detection of cancer, which contains hsa-miR-92a-3p, is provided. The cancer detection marker of the embodiment can be used for, for example, the determination of the presence/absence of contraction of cancer in a subject, the determination of prognostication of cancer in a subject and the determination of the presence/absence of recurrence of cancer in a subject, and the like. Further, the cancer detection marker can be also used for the selection of the type of therapeutic method or the type of drug to apply to a subject. Here, the therapeutic method or drug is for treatment of cancer. Or, the cancer detection marker can be also used for the detection of cancer cells in a sample, and the like.

Further, according to the third embodiment, a kit for the detection of cancer is provided.

The kit contains at least one type of nucleic acid selected from a group consisting of a primer set to amplify hsa-miR-92a-3p, an RT primer to reverse-transcribing hsa-miR-92a-3p, an EL primer to elongate hsa-miR-92a-3p and nucleic acid probes to detect hsa-miR-92a-3p.

The cancer detection kit of the embodiment can be used for, for example, the determination of the presence/absence of contraction of cancer in a subject, the determination of prognostication of cancer in a subject and the determination of the presence/absence of recurrence of cancer in a subject, and the like. Further, the kit of this embodiment can be also used for the selection of the type of therapeutic method or the type of drug to apply to a subject. Here, the therapeutic method or drug is for treatment of cancer. Or, the kit of the embodiment can be also used for the detection of a cancer cell in samples, and the like.

According to another embodiment, the cancer detection kit is provided as a composition for diagnosis for cancer or a diagnostic drug thereof containing at least one type of nucleic acid selected from a group consisting of a primer set to amplify hsa-miR-92a-3p, an RT primer to reverse-transcribing hsa-miR-92a-3p, an EL primer to elongate hsa-miR-92a-3p and nucleic acid probes to detect hsa-miR-92a-3p. Further, according to the embodiment, use of at least one kind of the nucleic acids described above in the production of the composition for the diagnosis of cancer or the diagnostic drug of cancer is also provided.

### [Examples]

Experiments carried out using the analytical methods or the kits of the embodiments will now be described.

Example 1. Quantification (the qRT-PCR method) of hsa-miR-92a-3p in serums of a normal person and serums of cancer patients

Serums prepared for the Examples were obtained from 16 samples of normal persons, 22 samples of breast cancer patients, 6 samples of colon cancer patients, 6 samples of stomach cancer patients, 3 samples of lung cancer patients, 6 samples of ovarian cancer patients, 11 samples of pancreatic cancer patients, 5 samples of bile duct cancer patient, 5 samples of esophagus cancer patients, 5 samples of liver cancer patients, 5 samples of brain tumor patients, 5 samples of bladder cancer patients, 5 samples of prostatic cancer patients, 5 samples of sarcoma patients, 5 samples of endometrial cancer patients and 5 samples of uterus sarcoma patients.

From each serum, RNA was extracted. The extraction was carried out using NucleoSpin (registered trademark) miRNA Plasma (product name, a product of TAKARA BIO Corporation).

Then, short chain RNA present in each extracted sample was reverse-transcribed and thus cDNA was synthesized and amplified. The synthesis was done by using TaqMan (registered trademark) Advanced miRNA cDNA Synthesis Kit (product name, a product of ThermoFischer Scientific Company) according to the instruction book of TaqMan (registered trademark) Advanced miRNA (a product name, a product of ThermoFischer Scientific Company).

Subsequently, TaqMan-PCR was carried out to measure the Ct value and quantify the miRNA by using of TaqMan (registered trademark) Fast Advanced Master Mix (a product name, a product of ThermoFischer Scientific Company) and TaqMan (registered trademark) Advanced miRNA ID: 477827_mir.

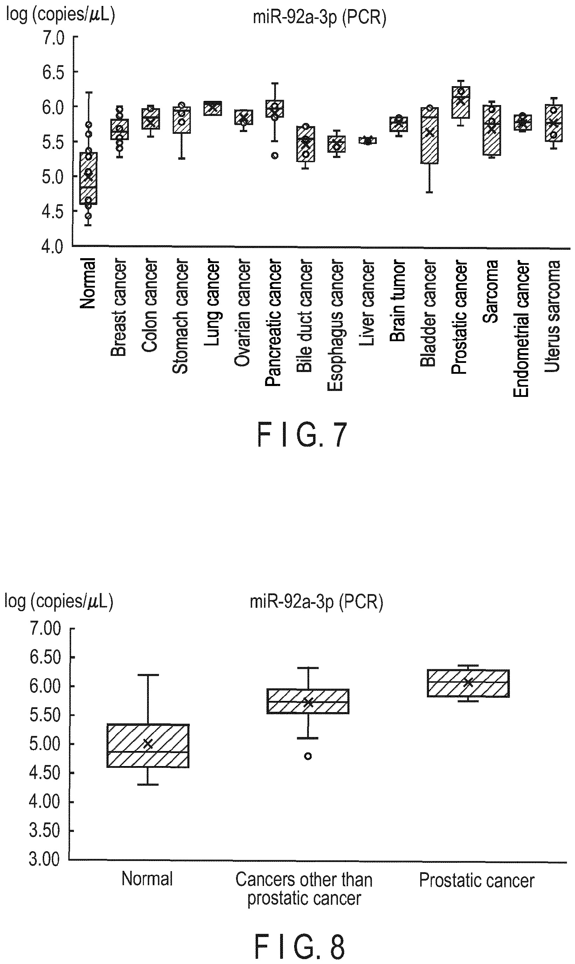

The results of the quantification are shown in FIGS. 7 and 8. The FIG. 7 shows the quantification value of hsa-miR-92a-3p in each of normal persons and patients with various types of cancers. The hsa-miR-92a-3p was distributed in the order of 10⁴ to 10⁵ copies in the normal persons, in the order of 10⁵ to 10⁶ copies in the cancer patients except for prostatic cancer, and in the order of 10⁶ copies in the prostatic cancer patients. These results indicate that a great amount of hsa-miR-92a-3p present in the serum was observed in the prostatic cancer patients.

FIG. 8 is a box plat diagram showing the results of the quantification in the normal persons, cancer patients except for prostatic cancer and prostatic cancer patients. The cancer patients were distributed over a higher value than that of the normal persons, and the prostatic cancer patients showed a further higher value than those of the other cancers.

Area-under-curves (AUC) which respectively separate the normal persons and cancer patients, prostatic cancer patients and normal persons plus patients with other cancers together, and prostatic cancer patients and patients with other cancer into categories, respectively, are shown in Table 3 below.

**Table 3**

| | AUC |
|---|---|
| Normal vs cancer | 0.88 |
| Prostatic cancer vs Normal + other cancers | 0.87 |
| Prostatic cancer vs other cancer | 0.85 |

The AUC which categorizes the normal persons and the cancer patients from each other was approximately 0.88, the AUC between the prostatic cancer patients and the normal persons plus the cancer patients together was approximately 0.87, and the AUC between the prostatic cancer patients and the other cancer patients was approximately 0.85.

Thus, it has been indicated that the miRNA of SEQ ID NO. 1, that is, hsa-miR-92a-3p is a high-performance marker that detects prostatic cancer while distinguishing from the normal persons and the other cancers. Further, it has been demonstrated that hsa-miR-92a-3p can be used as a marker that detects cancer patients while distinguishing from the normal persons.

Example 2. Quantification (the LAMP method) of hsa-miR-92a-3p in serum of normal persons and serum of cancer patients

Serums prepared for the Examples were obtained from 16 samples of normal persons, 5 samples of breast cancer patients, 5 samples of colon cancer patients, 5 samples of stomach cancer patients, 3 samples of lung cancer patients, 5 samples of ovarian cancer patients, 5 samples of pancreatic cancer patients, 5 samples of bile duct cancer patient, 5 samples of esophagus cancer patients, 5 samples of liver cancer patients, 5 samples of brain tumor patients, 5 samples of bladder cancer patients, 5 samples of prostatic cancer patients, 2 samples of sarcoma patients, 2 samples of endometrial cancer patients and 2 samples of uterus sarcoma patients.

The extraction of RNA was carried out by the same method as that of Example 1. 2 µL of each extracted sample was reverse-transcribed under conditions of 20µL of reaction in volume, ten minutes at 16°C, five minutes at 42°C and five minutes at 85°C. The composition of the reverse transcription reaction solution was 67 unit MultiScribe (registered trademark) Reverse Transcriptase (*), 1 × RT Buffer (*), 0.1 mM of dNTPs (*), 4 U of RNaseOUT (product name, a product of ThermoFischer Scientific Company), and 10nM of RT primer (RT primer, SEQ ID NO. 2). Those with a symbol "*" all included High-Capacity cDNA Reverse Transcription Kit (product name, a product of ThermoFischer Scientific Company).

To the reaction solution after the reverse transcription, 5 µL of an elongation liquid was added, and the elongation was carried out after two minutes at 95°C and then by 20 times of a cycle of 20 seconds at 95°C - 30 seconds at 59°C - 10 seconds at 72°C. The elongation liquid was 25 µL of an elongation reaction solution containing DeepVent(exo-)DNApolymerase (0.5 U, a product of New England Bio), which was prepared to have a final concentration in each of 0.2 × ThermoPol Buffer (with attachment of DeepVent(exo-)DNApolymerase), 0.2 mM of MgSO₄, 0.12 mM of dNTPs and 10 nM of EL primer (EL primer, SEQ ID NO. 3).

Then, 1 µL of the elongated product was LAMP-amplified under conditions of a reaction volume of 25 µL and 60 minutes at 65°C and at the same time, the rise time of the fluorescence intensity was measured. The LAMP liquid contained 8 U of Tin(exo-)LF DNApolymerase (Optigene), and 0.5 µL of EvaGreen (registered trademark) (a product of Biotium), and was prepared to have a final concentration in each of 20 mM of Tris-HCl (pH 8.0), 50 mM of KCl, 8 mM of MgSO₄, 10 mM of (NH₄)SO₄, 0.1% of Tween-20, 0.8 M of betaine, 1.4 mM of each of dNTPs, 1.6 µM of an FIP primer (SEQ ID NO. 8), 1.6 µM of a BIP primer (SEQ ID NO. 9) and 0.8 µM of an LB primer (SEQ ID NO. 10). With a real-time PCR device, the fluorescence intensity was measured over time, and a time period in which the level exceeds the threshold was measured.

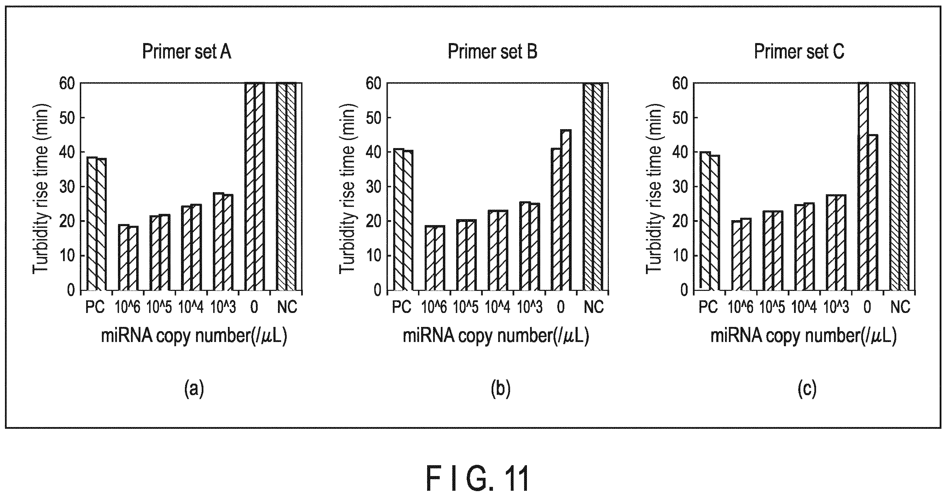

The synthetic RNA of SEQ ID NO. 1 was reverse-transcribed, elongated and LAMP-amplified for each of the cases of 0, 10³, 10⁴, 10⁵ and 10⁶ copies/µL using primers similar to those described above and the rise time of the fluorescence intensity was measured for each. From the results of these, an analytical curve of the number of copies of RNA of SEQ ID NO. 1 and the rise time was prepared for each (See FIG. 11, part (a)). Using the analytical curve, the number of copies of RNA was calculated from the rise time in each sample.

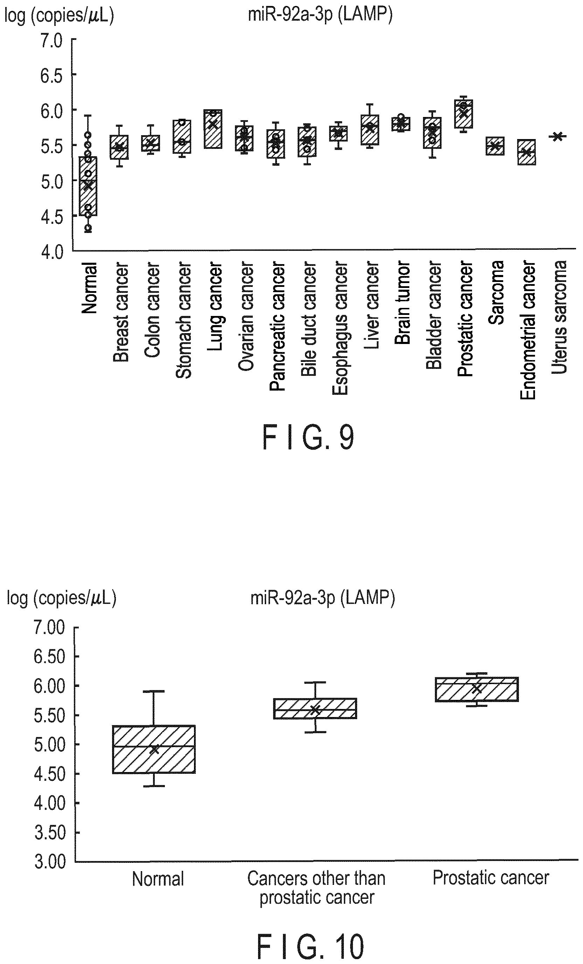

The results of the quantification are shown in FIGS. 9 and 10. FIG. 9 is a box plot diagram showing the results of the quantification value of the normal persons and patients with various types of cancers. The quantification value was distributed in the order of 10⁴ to 10⁵ copies in the normal persons, in the order of 10⁵ copies in the cancer patients except for prostatic cancer, and in the order of 10⁶ copies in the prostatic cancer patients. These results indicate that a great amount of hsa-miR-92a-3p present in the serum was observed in the prostatic cancer patients.

FIG. 10 is a box plot diagram showing the results of the quantification of the normal persons, the cancer patients except for prostatic cancer, and the prostatic cancer patients. The results indicates that the cancer patients were distributed over a higher value than that of the normal persons, and the prostatic cancer patients showed a further higher value than those of the other cancers.

Area-under-curves (AUC) which respectively separate the normal persons and cancer patients, prostatic cancer patients and normal persons plus patients with other cancers together, and prostatic cancer patients and patients with other cancer into categories, respectively, are shown in Table 4 below.

**Table 4**

| | AUC |
|---|---|
| Normal vs cancer | 0.92 |
| Prostatic cancer vs Normal + other cancers | 0.89 |
| Prostatic cancer vs other cancer | 0.87 |

The AUC which categorizes the normal persons and the cancer patients from each other was approximately 0.92, the AUC between the prostatic cancer patients and the normal persons plus the cancer patients together was approximately 0.89, and the AUC between the prostatic cancer patients and the other cancer patients was approximately 0.87.

Thus, it has been indicated that the miRNA of SEQ ID NO. 1, that is, hsa-miR-92a-3p is a high-performance marker that detects prostatic cancer while distinguishing from the normal persons and the other cancers. It has been also indicated that hsa-miR-92a-3p can be used as a marker for detecting cancer patients while distinguishing from the normal persons. Further, the results of the example which employed the LAMP method, it has been made clear that a higher separation ability is achieved than in the case of qRT-PCR used in Example 1.

### Example 3. Evaluation of specificity of primer sets

Four sets of primer sets each containing an RT primer, an EL primer, an FIP primer, a BIP primer and an LB primer were synthesized. With use of these, sequences and combinations of primers which could specifically elongate and amplify hsa-miR-92a-3p were evaluated. for each of the cases of 0, 10³, 10⁴, 10⁵ and 10⁶ copies/µL of the synthetic RNA of SEQ ID NO. 1 was elongated and LAMP-amplified using the four types of primer sets under the same conditions as those of Example 2. The amplification was carried out using an end point turbidimetry device (LT -16, a product of NIPPON GENE) and the rise time of the fluorescence intensity was measured for each. As a positive control, those obtained by artificially synthesizing elongation products corresponding to the sequences of the respective primer sets were synthesized for each case to be used.

Of the four sets, the results of the rise time of the turbidity of each of the primer sets A, B and C shown in Table 5 are shown in FIG. 11, parts (a), (c) and (b), respectively.

**Table 5**

| Primer set A | | |
|---|---|---|
| Primer | SEQ ID NO | Sequence (5'-3') |
| RT | 2 | GGAGGCGACACGAGTTCTACAGGCCG |
| EL | 3 | GGCTGTGCAGAGATAGGTGGTACGAAGCAGCCTTCTCGGCTCGGCTAT CTACGCGTTAAGCGGGGTATTGCACTTGTCC |
| FIP | 8 | GCCGAGAAGGCTGCTTCGTAGGCTGTGCAGAGATAGGTG |
| BIP | 9 | TCGGCTATCTACGCGTTAAGCGGGAGGCGACACGAGTTCT |
| LB | 10 | ACTTGTCCCGGCCTGT |

| Primer set B | | |
|---|---|---|
| Primer | SEQ ID NO | Sequence (5'-3') |
| RT | 4 | GGCGCCGAAACAATATTCCTACAGGCCG |
| EL | 5 | GATCTAGAAGGCCGCCAGTCGTTCAGCCTACGGCCGTTGTCATCCGTAG CAGGACGCTCAGGGTATTGCACTTGTCC |
| FIP | 12 | AACGGCCGTAGGCTGAACGGATCTAGAAGGCCGCCAGT |
| BIP | 13 | GTCATCCGTAGCAGGACGCTCAGGCGCCGAAACAATATTCCT |
| LB | 10 | ACTTGTCCCGGCCTGT |

| Primer set C | | |
|---|---|---|
| Primer | SEQ ID NO | Sequence (5'-3') |
| RT | 6 | CTGCAACGTTGAACATGCGACAGGCCG |
| EL | 7 | TTCGTGCAGATGGCTATGCGGCTAGAATACGGCACGACTGCTGATGTGT CTGATAGCGGCACGGGGTATTGCACTTGTCC |
| FIP | 14 | GCAGTCGTGCCGTATTCTAGCCCTTCGTGCAGATGGCTATGC |
| BIP | 15 | TGATGTGTCTGATAGCGGCACGCTGCAACGTTGAACATGCG |
| LB | 10 | ACTTGTCCCGGCCTGT |

In the case where the primer sets A to C is used, the miRNA concentration-dependency of the rise time of the turbidity was more excellent and nonspecific amplification when miRNA copy number was 0 was reduced as compared to the cases of the other primer sets. Especially, as to the primer set A, the miRNA concentration-dependency of the rise time of the turbidity was high, and the nonspecific amplification when miRNA copy number was 0 was not observed, thus exhibiting a particularly excellent result.

It has been suggested from the results described above that it is possible to specifically elongate and amplify the target miRNA by using the primer sets A to C. Thus, it has been suggested that the accuracy of the detection of prostate cancer is improved with use of the primer sets.

### Example 4. Examination of LB sequence

The elongation product for analytical curve, formed with the synthetic RNA, prepared in Example 2 was amplified by a method similar to that of Example 2 using an LB sequence of SEQ ID NO. 11 listed in Table 6 in place of the LB sequence of SEQ ID NO. 10.

**Table 6**

| Primer | SEQ ID NO | Sequence (5'-3') |
|---|---|---|
| LB | 11 | TTGTCCCGGCCTGTAGA |

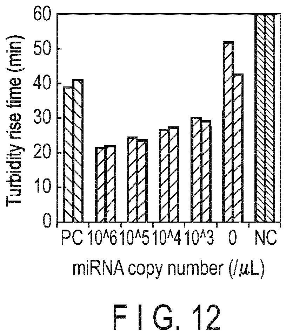

The results are shown in FIG. 12, which indicate that when using the LB primer of SEQ ID NO. 11 as well, the miRNA concentration-dependency of the rise time of the turbidity was excellent, and the nonspecific amplification obtained when the miRNA copy number was 0 was less. Therefore, it has been indicated the LB sequence of SEQ ID NO. 11 can also be used effectively in the LAMP system.

### Example 5. Electrochemical Detection

A primer solution containing the FIP primer and BIP primer (48 µM each) used in Example 2, and an LB primer (24 µM) was prepared. 100 nL of the primer solution was spotted on a silicone-made flow path packing (width × height: 1 mm × 1 mm) using a micro-dispenser. A DNA chip substrate (glass (0.8 mm)/titanium (500 nm)/gold (2,000 nm)) in which an electrode was patterned and the flow path packing were incorporated in a cassette, and thus a chip was manufactured.

Then, an LAMP reaction solution of the composition listed in Table 5 was prepared.

**Table 7 Composition of LAMP reaction solution**

| Ingredients | Final concentration |
|---|---|
| Tris-HCl (pH8.8) | 20 mM |
| KCl | 60 mM |
| MgSO₄ | 8 mM |
| (NH₄)₂SO₄ | 10 mM |
| Tween20 | 0.1 % |
| dNTPs | 1.4 mM each |
| Tin exo-DNA polymerase | 48 units (x3) |
| Betaine | 0.8 M |
| RuHex | 1 mM |
| Template | 1 µL |
| Amount of reaction solution | 60 µL |

1 µL of a template after the elongation used in Example 2 was added to the LAMP reaction solution, and electrochemical measurement was carried out under conditions indicated in Table 8.

**Table 8**

| Conditions for electrochemical measurement | |
|---|---|
| Items | Details |
| Measurement method | Linear Sweep Voltammetry (LSV) |
| Sweep potential | 0.1 to -0.4 V |
| Sweep rate | 0.5 V/s |
| Temperature | 65°C |

When the LAMP reaction was started, the reduction current value of ruthenium hexaamine (RuHex) began to increase. It is clear that the time at which the current increased was earlier as the amount of the elongation product present was greater, and therefore with use of the chip, the quantification can be detected.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the spirit of the inventions. The accompanying claims and their equivalents are intended to cover such forms or modifications as would fall within the scope and spirit of the inventions.

## Claims

1. An analytical method for determining presence/absence of contraction of prostatic cancer in a subject, comprising:
quantifying hsa-miR-92a-3p in a sample originated from the subject.

2. An analytical method for assisting determination of presence/absence of contraction of prostatic cancer in a subject, comprising:
quantifying hsa-miR-92a-3p in a sample originated from the subject.

3. The method of claim 1 or 2, wherein
the determination of the presence/absence of the contraction of prostatic cancer is carried out without using the result of detection or quantification of a marker other than hsa-miR-92a-3p.

4. The method of any one of claims 1 to 3, further comprising a determination step of determining that the subject contracts the prostatic cancer when an amount of hsa-miR-92a-3p present in the subject is greater than an amount of hsa-miR-92a-3p present in a control.

5. The method of any one of claims 1 to 4, wherein
the cancer including prostate cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer and uterus sarcoma.

6. The method of any one of claims 1 to 5, wherein
the cancer is prostate cancer, and the presence/absence of the contraction of prostatic cancer is determined as distinct from other cancers.

7. The method of claim 6, wherein
the other cancers including breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer and uterus sarcoma.

8. The method of any one of claims 1 to 7, wherein
the sample is serum.

9. The method of any one of claims 1 to 8, wherein
the quantification is carried out using at least one type of nucleic acid selected from a group consisting of a primer set to amplify hsa-miR-92a-3p (referred to as "the first primer set" hereinafter), a reverse-transcription primer to reverse-transcribe hsa-miR-92a-3p, an elongation primer to elongate hsa-miR-92a-3p and nucleic acid probes to detect hsa-miR-92a-3p.

10. The method of claim 9, wherein
the first primer set contain:
a FIP primer of SEQ ID NO. 8, a BIP primer of SEQ ID NO. 9 and an LB primer of SEQ ID NO. 10;
an FIP primer of SEQ ID NO. 8, a BIP primer of SEQ ID NO. 9 and an LB primer of SEQ ID NO. 11;
an FIP primer of SEQ ID NO. 12, a BIP primer of SEQ ID NO. 13 and an LB primer of SEQ ID NO. 10; or
an FIP primer of SEQ ID NO. 14, a BIP primer of SEQ ID NO. 15 and an LB primer of SEQ ID NO. 10;
and
the reverse transcription primer contains SEQ ID NO. 2 and the elongation primer contains SEQ ID NO. 3, or
the reverse transcription primer contains SEQ ID NO. 4 and the elongation primer contains SEQ ID NO. 5, or
the reverse transcription primer contains SEQ ID NO. 6, and the elongation primer contains SEQ ID NO. 7.

11. A kit for detecting cancer, containing at least one type of nucleic acid selected from a group comprising of a primer set to amplify hsa-miR-92a-3p (to be referred to as a "first primer set" hereinafter), a reverse transcription primer to reverse-transcribe hsa-miR-92a-3p, an elongation primer to elongate hsa-miR-92a-3p, and nucleic acid probes to detect hsa-miR-92a-3p.

12. The kit of claim 11, wherein
the cancer including prostate cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer and uterus sarcoma.

13. The kit of claim 12, wherein
the cancer is prostate cancer.

14. The kit of any one of claims 11 to 13, wherein
the first primer set contains:
an FIP primer of SEQ ID NO. 8, a BIP primer of SEQ ID NO. 9 and an LB primer of SEQ ID NO. 10,
an FIP primer of SEQ ID NO. 8, a BIP primer of SEQ ID NO. 9 and an LB primer of SEQ ID NO. 11,
an FIP primer of SEQ ID NO. 12, a BIP primer of SEQ ID NO. 13 and an LB primer of SEQ ID NO. 10, or
an FIP primer of SEQ ID NO. 14, a BIP primer of SEQ ID NO. 15 and an LB primer of SEQ ID NO. 10, and
the reverse transcription primer contains SEQ ID NO. 2, and the elongation primer contains SEQ ID NO. 3, or
the reverse transcription primer contains SEQ ID NO. 4, and the elongation primer contains SEQ ID NO. 5, or
the reverse transcription primer contains SEQ ID NO. 6 and the elongation primer contains SEQ ID NO. 7.

15. A marker for detecting cancer, comprising hsa-miR-92a-3p.

16. The marker of claim 15, wherein
the cancer including prostate cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer and uterus sarcoma.

17. The marker of claim 16, wherein
the cancer is prostate cancer.

## Amended claims

### Amended claims under Art. 19.1 PCT

1. (Amended) An analytical method for determining presence/absence of contraction of cancer in a subject, comprising: quantifying hsa-miR-92a-3p in a sample originated from the subject,
the cancer being prostatic cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer or uterus sarcoma.

2. (Amended) An analytical method for assisting determination of presence/absence of contraction of cancer in a subject, comprising: quantifying hsa-miR-92a-3p in a sample originated from the subject,
the cancer being prostatic cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer or uterus sarcoma.

3. The method of claim 1 or 2, wherein
the determination of the presence/absence of the contraction of prostatic cancer is carried out without using a result of detection or quantification of a marker other than hsa-miR-92a-3p.

4. The method of any one of claims 1 to 3, further comprising determining that the subject contracts the prostatic cancer when an amount of hsa-miR-92a-3p present in the subject is greater than an amount of hsa-miR-92a-3p present in a control.

5. (Amended) The method of any one of claims 1 to 4, wherein
the cancer is prostate cancer, and the presence/absence of the contraction of prostatic cancer is determined as distinct from other cancers.

6. (Amended) The method of claim 5, wherein the other cancers including breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer and uterus sarcoma.

7. (Amended) The method of any one of claims 1 to 6, wherein
the sample is serum.

8. (Amended) The method of any one of claims 1 to 7, wherein
the quantification is carried out using at least one type of nucleic acid selected from a group consisting of a primer set to amplify hsa-miR-92a-3p (referred to as "the first primer set" hereinafter), a reverse-transcription primer to reverse-transcribe hsa-miR-92a-3p, an elongation primer to elongate hsa-miR-92a-3p and nucleic acid probes to detect hsa-miR-92a-3p.

9. (Amended) The method of claim 8, wherein
the first primer set contain:
a FIP primer of SEQ ID NO. 8, a BIP primer of SEQ ID NO. 9 and an LB primer of SEQ ID NO. 10;
an FIP primer of SEQ ID NO. 8, a BIP primer of SEQ ID NO. 9 and an LB primer of SEQ ID NO. 11;
an FIP primer of SEQ ID NO. 12, a BIP primer of SEQ ID NO. 13 and an LB primer of SEQ ID NO. 10; or
an FIP primer of SEQ ID NO. 14, a BIP primer of SEQ ID NO. 15 and an LB primer of SEQ ID NO. 10;
and
the reverse transcription primer contains SEQ ID NO. 2 and the elongation primer contains SEQ ID NO. 3, or
the reverse transcription primer contains SEQ ID NO. 4 and the elongation primer contains SEQ ID NO. 5, or
the reverse transcription primer contains SEQ ID NO. 6, and the elongation primer contains SEQ ID NO. 7.

10. (Amended) A kit for detecting cancer, containing a primer set to amplify hsa-miR-92a-3p (to be referred to as a "first primer set" hereinafter), a reverse transcription primer to reverse-transcribe hsa-miR-92a-3p, an elongation primer to elongate hsa-miR-92a-3p, and at least one type of nucleic acid selected from a group comprising nucleic acid probes to detect hsa-miR-92a-3p,
the cancer being prostatic cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer or uterus sarcoma.

11. (Amended) The kit of claim 10, wherein
the cancer is prostatic cancer.

12. (Amended) The kit of claim 10 or 11, wherein
the first primer set contains:
an FIP primer of SEQ ID NO. 8, a BIP primer of SEQ ID NO. 9 and an LB primer of SEQ ID NO. 10,
an FIP primer of SEQ ID NO. 8, a BIP primer of SEQ ID NO. 9 and an LB primer of SEQ ID NO. 11,
an FIP primer of SEQ ID NO. 12, a BIP primer of SEQ ID NO. 13 and an LB primer of SEQ ID NO. 10, or
an FIP primer of SEQ ID NO. 14, a BIP primer of SEQ ID NO. 15 and an LB primer of SEQ ID NO. 10, and
the reverse transcription primer contains SEQ ID NO. 2, and the elongation primer contains SEQ ID NO. 3, or
the reverse transcription primer contains SEQ ID NO. 4, and the elongation primer contains SEQ ID NO. 5, or
the reverse transcription primer contains SEQ ID NO. 6 and the elongation primer contains SEQ ID NO. 7.

13. (Amended) A marker for detecting cancer, comprising hsa-miR-92a-3p,
the cancer being prostatic cancer, breast cancer, colon cancer, stomach cancer, lung cancer, ovarian cancer, pancreatic cancer, bile duct cancer, esophagus cancer, liver cancer, brain tumor, bladder cancer, sarcoma, endometrial cancer or uterus sarcoma.

14. (Amended) The marker of claim 13, wherein
the cancer is prostate cancer.

15. (Deleted)

16. (Deleted)

17. (Deleted)
